# EUROPEAN PATENT APPLICATION

(11) **EP 4 389 208 A1**
(43) Date of publication of application: **26.06.2024**
(21) Application number: 23218714.6
(22) Date of filing: 20.12.2023
(51) Int. Cl.: A61N 5/10

(54) **GANTRY DRIVE**

(30) Priority: 23.12.2022 GB 202219661
(71) Applicant: Elekta Limited, Crawley, Sussex RH10 9RR (GB)
(72) Inventor: WIBERG, Kristian, Sussex, RH10 9RR (GB); MINOZ, Alain, Sussex, RH10 9RR (GB); EKETJÄLL, Martin, Sussex, RH10 9RR (GB)
(74) Representative: Woodhill, Matthew James

(57) **Abstract**

There is provided a radiotherapy apparatus comprising a rotatable ring-shaped gantry and a direct drive mechanism, wherein the direct drive mechanism is configured to cause rotation of the gantry. There is also provided a method of rotating a gantry of a radiotherapy apparatus. The method comprises causing rotation of the gantry using a direct drive mechanism.

## Description

This disclosure relates to rotatable gantries of radiotherapy systems, and specifically driving mechanisms for such gantries.

### Background

Radiotherapy can be described as the use of ionising radiation, such as X-rays, to treat a human or animal body. Radiotherapy is commonly used to treat tumours within the body of a patient or subject. In such treatments, ionising radiation is used to irradiate, and thus destroy or damage, cells which form part of the tumour.

A radiotherapy device typically comprises an annular gantry which supports a beam generation system, or other source of radiation, which is rotatable around a patient. For example, for a linear accelerator (linac) device, the beam generation system may comprise a source of radio frequency energy, a source of electrons, an accelerating waveguide, beam shaping apparatus, etc.

In conventional radiotherapy devices, rotation of the gantry is driven by a motor connected to a transmission system which causes the rotation of the gantry. Typically, small rollers at the base of the rotatable gantry are positioned to be in contact with the gantry, such that rotation of the rollers (driven by the motor and transmission system) causes rotation of the gantry.

Since the gantry supports various radiotherapy components such as those described above, a high torque is required to rotate the gantry. To achieve the high torque, a high gear ratio between the rollers and the gantry is required, which means that the rollers are required to have a very small diameter in relation to the diameter of the gantry. Therefore, the rollers must rotate at very high speeds in order to rotate the gantry at the required operation speed for carrying out imaging or delivering radiotherapy treatment. The required operation speed for the gantry may be above 20 revolutions per minute (rpm) or higher, which necessitates that the rollers rotate at 120 rpm or higher. The high rotation speed of the rollers can produce high levels of noise which can be discomforting and disconcerting for the patient. Moreover, the high rotation speeds at which the rollers and other components operate causes wear and tear and results in shorter lifespans, meaning that the radiotherapy device is frequently out of service whilst these components are replaced.

The present invention seeks to address these and other disadvantages encountered in the prior art.

### Summary

According to a first aspect of the present disclosure, there is provided a radiotherapy apparatus comprising a rotatable ring-shaped gantry and a direct drive mechanism, wherein the direct drive mechanism is configured to cause rotation of the gantry. The direct drive mechanism is configured to transmit torque directly to the gantry without intermediate transmission components such as gears, belts, pulleys, or other means intended for transmitting torque from a motor to a load.

In some embodiments, the radiotherapy apparatus further comprises a base, and the gantry is configured to rotate relative to the base. The direct drive mechanism comprises a rotary portion fixed to the gantry and a stationary portion fixed to the base.

In some embodiments, the direct drive mechanism comprises a ring-shaped torque motor. The torque motor may comprises a rotor and a stator, wherein the rotor is coaxial with and mounted to the gantry, and wherein the stator is mounted to the base.

In other embodiments, the direct drive mechanism comprises a curved linear motor. The curvature of the curved linear motor may correspond to the curvature of the gantry. The curved linear motor comprises a rotor and a stator, wherein the rotor is mounted to the gantry and wherein the stator is mounted to the base.

In some embodiments, the gantry comprises a drum and an encoder strip wound around the drum of the gantry. In some embodiments, the radiotherapy device further comprises at least one encoder sensor and a rotational measurement system. The encoder sensor is configured to read the encoder strip and the rotational measurement system is configured to measure rotational position of the gantry based on the reading of the encoder sensor. In some embodiments, two or more encoder sensors are used and are configured to read the encoder strip. The encoder sensors may be fixed to the base or the stationary portion of the direct drive mechanism.

According to a second aspect of the present disclosure, there is provided a direct drive mechanism for a radiotherapy apparatus. The direct drive mechanism is configured to drive rotation of a rotatable gantry of the radiotherapy apparatus. In some embodiments, the direct drive mechanism comprises a torque motor. In other embodiments, the direct drive mechanism comprises a curved linear motor.

According to a third aspect of the present disclosure, a method of rotating a gantry of a radiotherapy apparatus is provided. The method comprises causing rotation of the gantry using a direct drive mechanism. In some embodiments, the gantry comprises a drum and an encoder strip wound around the drum, and the method comprises determining a rotational position of the gantry by reading the encoder strip using an encoder sensor. In some embodiments, two or more encoder sensors are used to read the encoder strip to determine the rotational position of the gantry. In some embodiments, the method further comprises controlling rotation of the gantry based on the determined of the rotational position of the gantry. The method may also further comprise controlling a radiotherapy treatment based on the determined rotational position of the gantry.

According to a fourth aspect of the present disclosure, there is provided a computer-readable medium carrying instructions that, when executed by one or more processors, cause the one or more processors to carry out any one of the methods disclosed herein.

### Brief description of the drawings

Specific embodiments are now described, by way of example only, with reference to the drawings, in which:
Figure 1 depicts a radiotherapy device or apparatus according to the present disclosure;
Figure 2 depicts a known radiotherapy device;
Figure 3a depicts a head-on view of a radiotherapy device according to some embodiments of the present disclosure;
Figure 3b depicts a side-on view of the radiotherapy device of Figure 3a;
Figure 3c depicts a head-on view of a radiotherapy device according to some embodiments of the present disclosure;
Figure 3d depicts a side-on view of the radiotherapy device of Figure 3c;
Figure 4a depicts a head-on view of a radiotherapy device according to some embodiments of the present disclosure;
Figure 4b depicts a side-on view of the radiotherapy device of Figure 4a;
Figure 4c depicts a head-on view of a radiotherapy device according to some embodiments of the present disclosure;
Figure 4d depicts a side-on view of the radiotherapy device of Figure 4a;
Figure 5 depicts a radiotherapy device according to some embodiments of the present disclosure;
Figure 6 depicts a flowchart of a method according to some embodiments of the present disclosure;
Figure 7 depicts a block diagram a radiotherapy system according to some embodiments of the present disclosure; and
Figure 8 depicts a computer program product arranged to instruct a computer to carry out the method of the present disclosure.

### Detailed Description

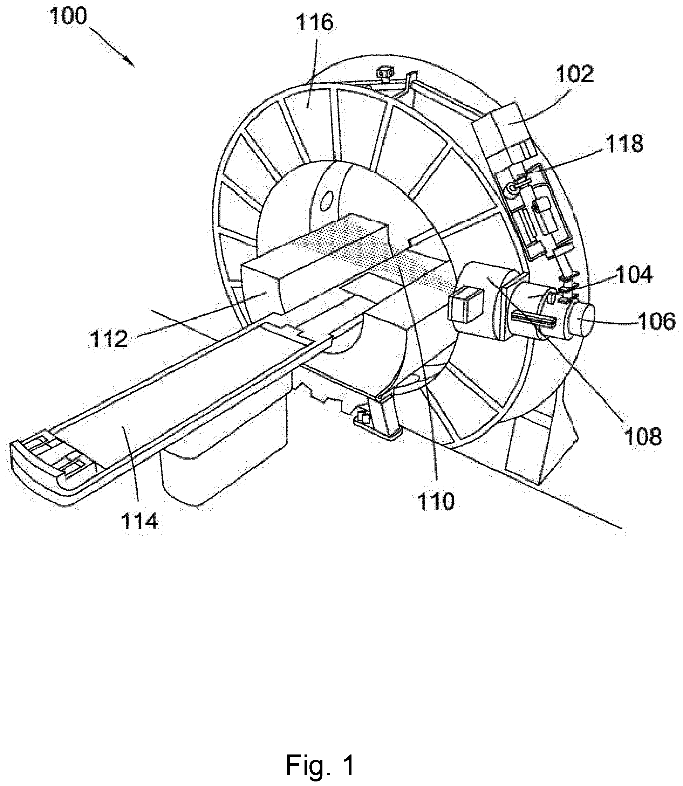

Figure 1 depicts a radiotherapy device suitable for delivering, and configured to deliver, a beam of radiation to a patient during radiotherapy treatment. The device and its constituent components will be described generally for the purpose of providing useful accompanying information for the present invention. The device depicted in figure 1 is in accordance with the present disclosure and is suitable for use with the disclosed systems and apparatuses. While the device in figure 1 is an MR-linac, the implementations of the present disclosure may be any radiotherapy device, for example a linac device.

The device 100 depicted in figure 1 is an MR-linac. The device 100 comprises both MR imaging apparatus 112 and radiotherapy (RT) apparatus which may comprise a linac device. The MR imaging apparatus 112 is shown in cross-section in the diagram. In operation, the MR scanner produces MR images of the patient, and the linac device produces and shapes a beam of radiation and directs it toward a target region within a patient's body in accordance with a radiotherapy treatment plan. The depicted device does not have the usual 'housing' which would cover the MR imaging apparatus 112 and RT apparatus in a commercial setting such as a hospital.

The MR-linac device depicted in figure 1 comprises a source of radiofrequency waves 102, a waveguide 104, a source of electrons 106, a source of radiation 106, a collimator 108 such as a multi-leaf collimator configured to collimate and shape the beam, MR imaging apparatus 112, and a patient support surface 114. In use, the device would also comprise a housing (not shown) which, together with the ring-shaped gantry, defines a bore. The moveable support surface 114 can be used to move a patient, or other subject, into the bore when an MR scan and/or when radiotherapy is to commence. The MR imaging apparatus 112, RT apparatus, and a subject support surface actuator are communicatively coupled to a controller or processor. The controller is also communicatively coupled to a memory device comprising computer-executable instructions which may be executed by the controller.

The RT apparatus comprises a source of radiation and a radiation detector (not shown). Typically, the radiation detector is positioned diametrically opposed to the radiation source. The radiation detector is suitable for, and configured to, produce radiation intensity data. In particular, the radiation detector is positioned and configured to detect the intensity of radiation which has passed through the subject. The radiation detector may also be described as radiation detecting means, and may form part of a portal imaging system.

The radiation source may comprise a beam generation system. For a linac, the beam generation system may comprise a source of RF energy 102, an electron gun 106, and a waveguide 104. The radiation source is attached to the rotatable gantry 116 so as to rotate with the gantry 116. In this way, the radiation source is rotatable around the patient so that the treatment beam 110 can be applied from different angles around the gantry 116. In a preferred implementation, the gantry is continuously rotatable. In other words, the gantry can be rotated by 360 degrees around the patient, and in fact can continue to be rotated past 360 degrees. The gantry may be ring-shaped. In other words, the gantry may be a ring-gantry.

The source 102 of radiofrequency waves, such as a magnetron, is configured to produce radiofrequency waves. The source 102 of radiofrequency waves is coupled to the waveguide 104 via circulator 118, and is configured to pulse radiofrequency waves into the waveguide 104. Radiofrequency waves may pass from the source 102 of radiofrequency waves through an RF input window and into an RF input connecting pipe or tube. A source of electrons 106, such as an electron gun, is also coupled to the waveguide 104 and is configured to inject electrons into the waveguide 104. In the electron gun 106, electrons are thermionically emitted from a cathode filament as the filament is heated. The temperature of the filament controls the number of electrons injected. The injection of electrons into the waveguide 104 is synchronised with the pumping of the radiofrequency waves into the waveguide 104. The design and operation of the radiofrequency wave source 102, electron source and the waveguide 104 is such that the radiofrequency waves accelerate the electrons to very high energies as the electrons propagate through the waveguide 104.

The design of the waveguide 104 depends on whether the linac accelerates the electrons using a standing wave or travelling wave, though the waveguide typically comprises a series of cells or cavities, each cavity connected by a hole or 'iris' through which the electron beam may pass. The cavities are coupled in order that a suitable electric field pattern is produced which accelerates electrons propagating through the waveguide 104. As the electrons are accelerated in the waveguide 104, the electron beam path is controlled by a suitable arrangement of steering magnets, or steering coils, which surround the waveguide 104. The arrangement of steering magnets may comprise, for example, two sets of quadrupole magnets.

Once the electrons have been accelerated, they may pass into a flight tube. The flight tube may be connected to the waveguide by a connecting tube. This connecting tube or connecting structure may be called a drift tube. The electrons travel toward a heavy metal target which may comprise, for example, tungsten. Whilst the electrons travel through the flight tube, an arrangement of focusing magnets act to direct and focus the beam on the target.

To ensure that propagation of the electrons is not impeded as the electron beam travels toward the target, the waveguide 104 is evacuated using a vacuum system comprising a vacuum pump or an arrangement of vacuum pumps. The pump system is capable of producing ultra-high vacuum (UHV) conditions in the waveguide 104 and in the flight tube. The vacuum system also ensures UHV conditions in the electron gun. Electrons can be accelerated to speeds approaching the speed of light in the evacuated waveguide 104.

The source of radiation is configured to direct a beam 110 of therapeutic radiation toward a patient positioned on the patient support surface 114. The source of radiation may comprise a heavy metal target toward which the high energy electrons exiting the waveguide are directed. When the electrons strike the target, X-rays are produced in a variety of directions. A primary collimator may block X-rays travelling in certain directions and pass only forward travelling X-rays to produce a treatment beam 110. The X-rays may be filtered and may pass through one or more ion chambers for dose measuring. The beam can be shaped in various ways by beam-shaping apparatus, for example by using a multi-leaf collimator 108, before it passes into the patient as part of radiotherapy treatment.

In some implementations, the source of radiation is configured to emit either an X-ray beam or an electron particle beam. Such implementations allow the device to provide electron beam therapy, i.e. a type of external beam therapy where electrons, rather than X-rays, are directed toward the target region. It is possible to 'swap' between a first mode in which X-rays are emitted and a second mode in which electrons are emitted by adjusting the components of the linac. In essence, it is possible to swap between the first and second mode by moving the heavy metal target in or out of the electron beam path and replacing it with a so-called 'electron window'. The electron window is substantially transparent to electrons and allows electrons to exit the flight tube.

The subject or patient support surface 114 is configured to move between a first position substantially outside the bore, and a second position substantially inside the bore. In the first position, a patient or subject can mount the patient support surface. The support surface 114, and patient, can then be moved inside the bore, to the second position, in order for the patient to be imaged by the MR imaging apparatus 112 and/or imaged or treated using the RT apparatus. The movement of the patient support surface is effected and controlled by a subject support surface actuator, which may be described as an actuation mechanism. The actuation mechanism is configured to move the subject support surface in a direction parallel to, and defined by, the central axis of the bore. The terms subject and patient are used interchangeably herein such that the subject support surface can also be described as a patient support surface. The subject support surface may also be referred to as a moveable or adjustable couch or table.

The radiotherapy apparatus / device depicted in figure 1 also comprises MR imaging apparatus 112. The MR imaging apparatus 112 is configured to obtain images of a subject positioned, i.e. located, on the subject support surface 114. The MR imaging apparatus 112 may also be referred to as the MR imager. The MR imaging apparatus 112 may be a conventional MR imaging apparatus operating in a known manner to obtain MR data, for example MR images. The skilled person will appreciate that such a MR imaging apparatus 112 may comprise a primary magnet, one or more gradient coils, one or more receive coils, and an RF pulse applicator. The operation of the MR imaging apparatus is controlled by the controller.

The controller is a computer, processor, or other processing apparatus. The controller may be formed by several discrete processors; for example, the controller may comprise an MR imaging apparatus processor, which controls the MR imaging apparatus 110; an RT apparatus processor, which controls the operation of the RT apparatus; and a subject support surface processor which controls the operation and actuation of the subject support surface. The controller is communicatively coupled to a memory, e.g. a computer readable medium.

The linac device also comprises several other components and systems as will be understood by the skilled person. For example, in order to ensure the linac does not leak radiation, appropriate shielding is also provided.

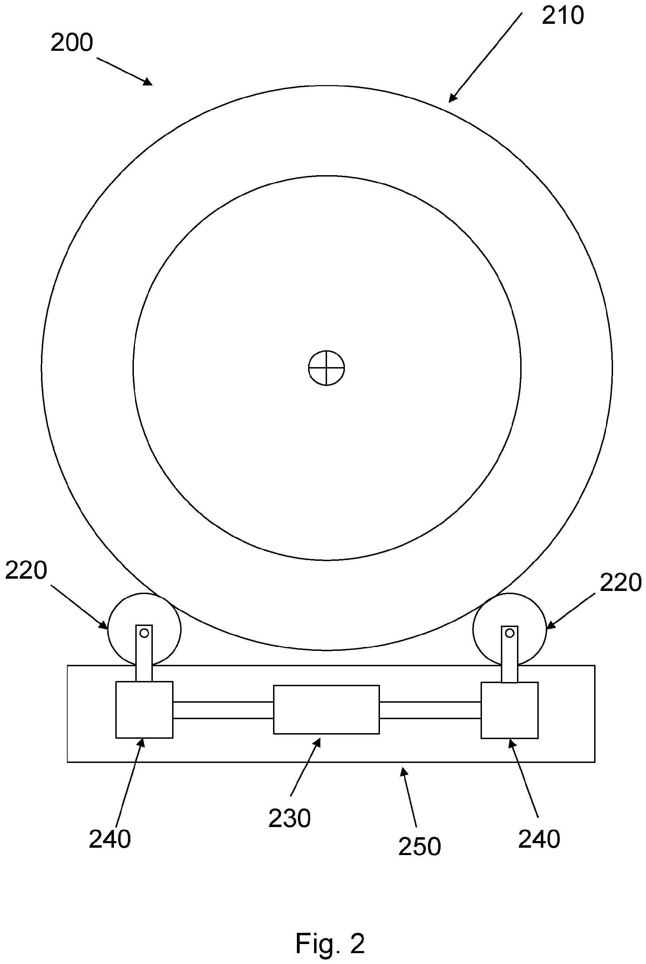

Figure 2 depicts a head-on view (i.e. looking directly through the bore along the axis of rotation of the gantry, i.e. along the axial direction) of a conventional radiotherapy device 200 that is known in the art. The device 200 comprises a ring-shaped (i.e. annular) gantry 210 that is configured to rotate about an axis of rotation passing through the centre of the gantry (indicated in the figure by the small circle with a cross through its centre). The radiotherapy device further comprises a base 250. The gantry is supported above the base by one or more rollers 220, such that the gantry 210 rests on the rollers 220. The rollers are configured to rotate and cause rotation of the gantry 210 about the gantry's axis of rotation. The rollers are driven by a drive mechanism comprising one or more motors 230 and one or more transmission mechanisms 240. Typically, each roller 220 may have a respective motor and transmission mechanism, or multiple rollers may share the same motor and/or transmission mechanism. In the example of a known device as depicted in Figure 2, the device comprises a single motor 230 driving respective transmission mechanisms 240 for each roller.

The transmission mechanisms 240 typically comprise any one or more of gears, belts, pulleys, or any other suitable components that are capable of transmitting rotary motion from the motor to the rollers 220.

Whilst not shown in the figure, conventional gantries of radiotherapy devices support multiple components for radiotherapy treatment and imaging. Supporting numerous radiotherapy components causes the gantry to be very heavy, and thus a high torque is required to cause rotation of the gantry about the rotation axis. In conventional devices, the high torque is typically produced at least in part by the high gear ratio between the rollers 240 and the gantry 210. As illustrated in Figure 2, the rollers typically have a much smaller diameter than the gantry. For example, conventional gantries typically have a diameter of the order of metres, whilst the rollers typically have a diameter of 30cm.

The high gear ratio required to produce enough torque to cause rotation of the gantry at the required speed results in a very high operational rotation speed of the rollers. Conventional gantries are required to rotate at a rate of 3 - 4rpm rpm when imaging and/or delivering radiotherapy treatment to a patient, which means that the rollers must operate at up to 25rom rpm. However, to reduce the patient throughput time for radiotherapy treatment, more recent conventional radiotherapy apparatuses have gantries that are required to rotate as a much faster rate - up to 20rpm. This means that the same rollers would be required to spin at up to 120rpm in order to deliver the required gantry rotation speed.

The high operating speeds leads to increased wear and tear on the various driving components (i.e. the motors 230, the transmission mechanisms 240 and the rollers 220), which means these components have a shorter lifespan and must be replaced more regularly. Additionally, the high operating speed produces high levels of noise which can be discomforting for the patient lying in the bore of the gantry.

Embodiments of the present disclosure seek to address these and other problems encountered in the prior art.

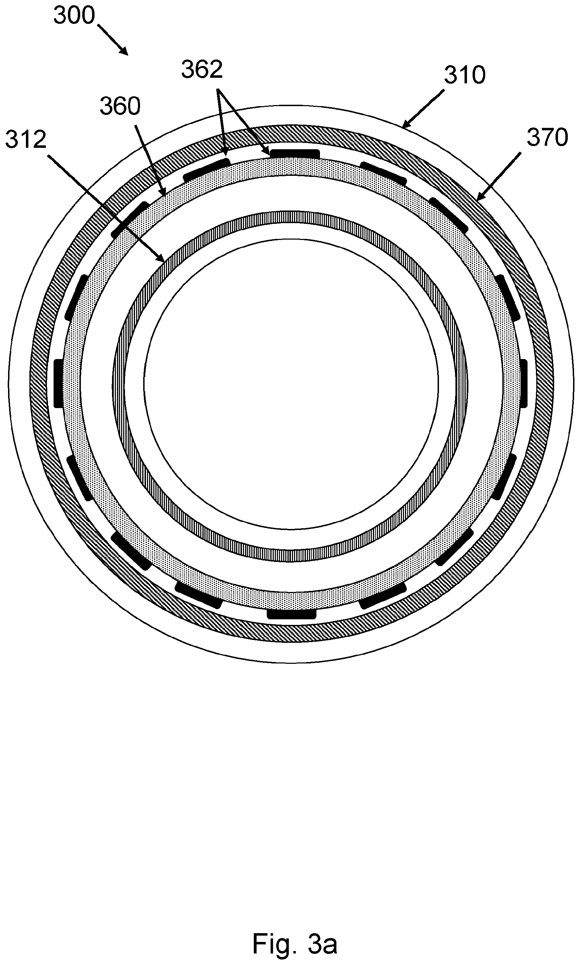

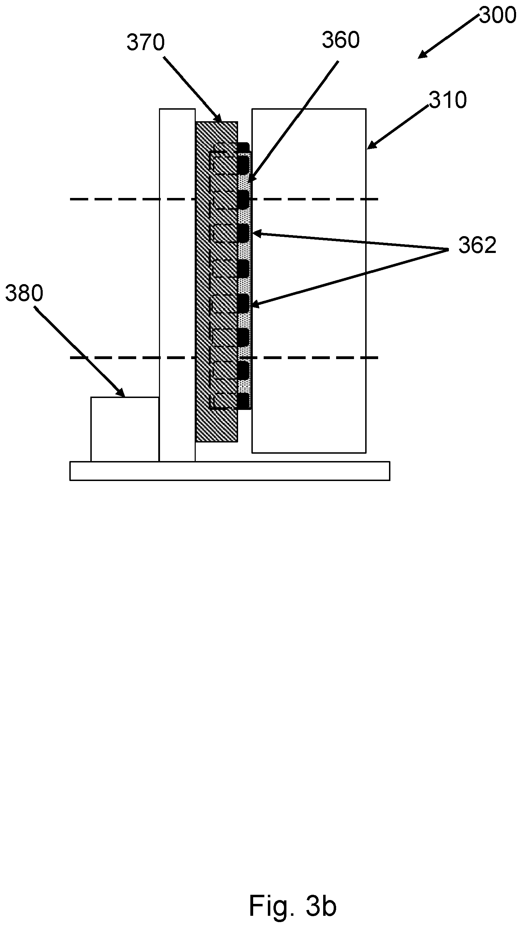

Referring now to Figure 3a and 3b, a rotatable gantry 310 of a radiotherapy device 300 according to one embodiment of the present disclosure is depicted. Figure 3a depicts a view of the gantry 310 along the longitudinal or axial direction (i.e. looking along the line of the axis of rotation). Figure 3b depicts the same gantry 310 from a side-on view, such that the axis of rotation runs horizontally through the drawing. Dashed lines in Figure 3b indicated the bore of the gantry, which is the bore of the radiotherapy device through which a patient is received for imaging or radiotherapy treatment. The gantry 310 optionally mounted to a stationary part of the radiotherapy device (not shown) via a central bearing 312 that is concentric with the gantry. In these examples, the gantry is suspended as a cantilever on the bearing 312. The bearing allows the gantry to rotate freely with respect to the stationary part of the radiotherapy apparatus, whilst the drive mechanism for rotating the gantry is described below. In other examples, the radiotherapy device may optionally further comprise a base (not shown) and one or more rollers (not shown) upon which rests the gantry. In such embodiments where the gantry 310 rests on one or more such rollers, the rollers may facilitate rotation of the gantry, however the rollers do not directly cause the rotation of the gantry. Unlike conventional devices as described above, radiotherapy devices according to the present disclosure do not require motors (such as motor 230) or transmission systems (such as mechanism 240) to cause rotation of the gantry.

Instead, the radiotherapy device of Figure 3a comprises a direct drive mechanism configured to cause rotation of the gantry without any intermediate transmission components (such as gears, belts, pulleys, worm gears, or the like). Instead, the direct drive mechanism is capable of transmitting torque generated using electromagnetic systems directly to the gantry 310, eliminating the need for intermediate transmission mechanisms that would otherwise be needed to transmit torque from a motor to the gantry. In this way, the elimination of transmission mechanisms enables a simple system for causing rotation of the gantry. Additionally, since no gear reduction is required, the use of a direct drive mechanism avoids the need for high-speed transmission components, meaning that fewer moving parts are required and which provides a durable, quiet and smooth system for rotating a gantry of a radiotherapy device. As discussed below, embodiments of the present disclosure may use brushless direct drive mechanisms, such as brushless torque motors or brushless linear motors. Such mechanisms do not require physical contact between the rotor and stator and therefore also lead to quieter and more durable systems, since there is no mechanical wear on either the rotor or stator caused by such contact.

In the particular embodiment depicted in Figure 3a and Figure 3b, the direct drive mechanism comprises a ring-shaped torque motor which includes a rotor 360 and a stator 370. The rotor 360 is mounted directly to the gantry such that rotation of the gantry 310 results from torque applied to the rotor by the stator. In some examples, the rotor 360 may be fixed to the gantry 310 using screws, bolts, or any other suitable fastening means apparent to the skilled person.

As can be seen in Figure 3a, the rotor 360 is ring-shaped and is concentric with the gantry. The diameter of bore of the rotor is at least as large as the diameter of the bore of the gantry 310, such that when in use, a patient may be positioned within the bore. The rotor comprises a plurality of magnets 362 spaced along the outward radial face of the rotor ring. The magnets therefore face towards the stator 370 (described below).

The torque motor further comprises a ring-shaped stator 370 which is not fixed to or in physical contact with the gantry 310 or rotor 360. Instead, as depicted in Figure 3b, the stator 370 is fixed to a stationary part of the device 300 (i.e. a part of the radiotherapy device that does not rotate with the gantry). The stator 370 is concentric with the gantry 310 and the rotor 360. In the particular embodiment illustrated in Figure 3a and 3b, the stator has a bore which is larger than the diameter of the rotor, such that the stator 370 is positioned to surround the rotor 360. As can be seen in Figure 3b, the stator 370 may at least partially overlap with the rotor 360 along the longitudinal/axial direction (parallel to the axis of rotation), meaning that, in the radial direction (perpendicular to the axis of rotation), the rotor and stator are adjacent. In preferable embodiments, the stator 370 completely overlaps with the rotor 360 so as to completely overlap the magnets 362 on the rotor.

The stator 370 may comprise a plurality of coils configured to carry electrical current in order to generate a torque in the rotor to cause rotation of the gantry 310. Since the ring-shaped rotor and stator have large diameters (the diameters being of the order of metres, similar to the diameter of the gantry), it is possible to generate and provide high levels of torque, which is required in order to cause rotation of the heavy gantry 310.

In the example depicted in Figure 3a, the bearing has a smaller diameter than the ring-shaped torque motor (made up of rotor 360 and stator 370) and is therefore positioned radially inside the torque motor. However it would be appreciated that in other examples, the bearing may have a larger diameter than the torque motor and therefore surrounds the torque motor. Further still, in some examples, the bearing may be fixed to the components of the torque motor itself. For example, the rotor 360 and stator 370 may each be fixed to separate components of the bearing 312 to allow the rotor to rotate with respect to the stator. In these examples, since the rotor is fixed to the gantry, the weight of the gantry is supported as a cantilever by the bearing via the bearings connection to the rotor and the stator. The bearing is not shown in Figure 3b since in the illustrated example, the bearing is positioned inside the torque motor and thus cannot be seen from the perspective shown in Figure 3b.

As can be seen in Figure 3b, in some embodiments the radiotherapy device further comprises a power supply unit 380 connected to the stator 370 and configured to supply electrical current to the coils of the stator. As would be understood by the skilled person, the supply of electrical current to the stator coils generates a magnetic field in those coils. The interaction of the magnetic fields produced by the coils and the magnetic fields produced by the magnets 362 on the rotor induces a torque in the rotor which causes rotation of the rotor and the gantry. The power supply 380 may further comprise a controller configured to control the supply of electrical current to the stator 370. The controller can control when and how much electrical power is supplied to the stator 370, so as to control gantry rotation position and the speed of rotation.

Whilst the specific embodiment depicted in Figures 3a and 3b depict a stator 370 positioned around the outside of the rotor 360 (and as such the magnets of the rotor are positioned on the outward-facing radial face of the rotor), it would be appreciated that the same result can be achieved with a stator 370 positioned inside the rotor 360. Thus, in some embodiments, the stator 370 has a smaller diameter than the rotor 360 and thus the rotor surrounds the stator. In these embodiments, the magnets 362 are positioned on the inward radial face of the rotor 362 so as to face towards the stator on the inside of the rotor. In either case, the rotor and stator overlap along the axial direction and are adjacent along the radial direction so as to generate torque in the rotor as described above.

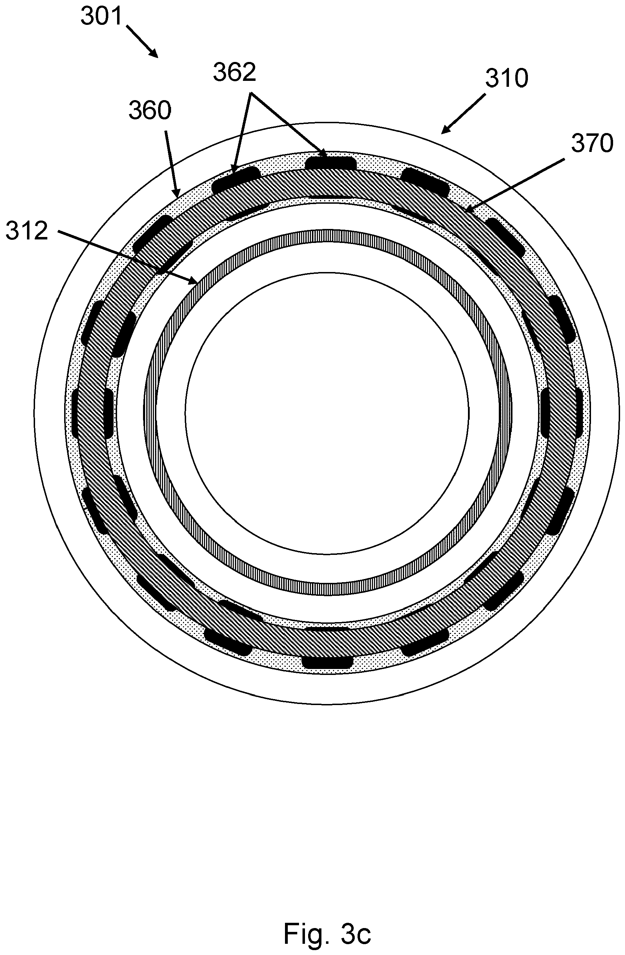

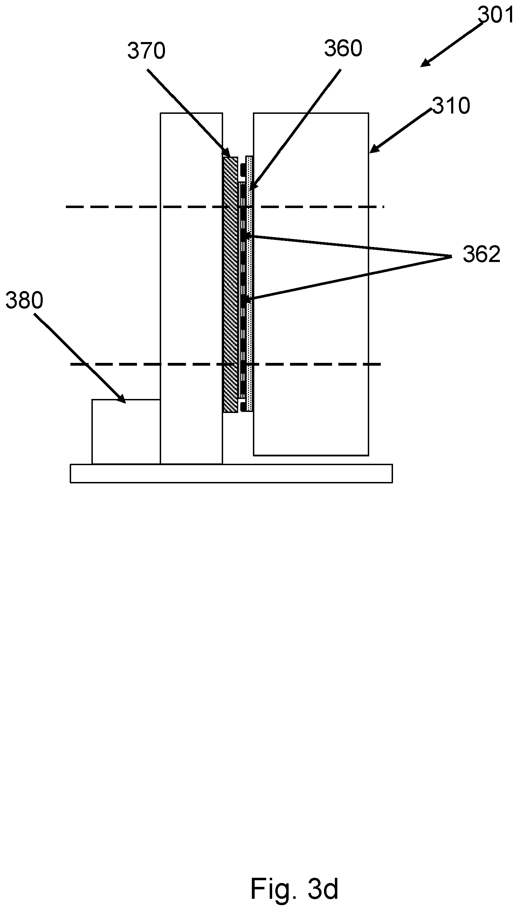

Referring now to Figure 3c and 3d, a radiotherapy device 301 according to another embodiment of the present disclosure is depicted. The radiotherapy device 301 is very similar to the device 300 depicted in Figures 3a and 3b and comprises a ring-shaped gantry 310, a ring-shaped rotor 360 mounted to the gantry, and a ring-shaped stator 370 fixed to a stationary part of the gantry, such as a base of the device. In some examples, the radiotherapy device 302 may further comprise a central bearing 312 for supporting the gantry as described above in relation to Figure 3a and 3b. In other examples, the radiotherapy device may be supported by rollers that facilitate rotation of the gantry but do not directly cause the rotation of the gantry.

Radiotherapy device 300 described above in relation to Figures 3a and 3b comprises magnets positioned on an outward (or inward) radial face of the rotor 360, and the stator 370 and rotor 360 overlap one another in the longitudinal/axial direction. This means that a magnetic flux path between the magnets 362 of the rotor and the coils of the stator is perpendicular to the axis of rotation, i.e. along radial direction. As such, the arrangement of the rotor and stator in the embodiment illustrated in Figure 3a and 3b may be referred to as a radial flux torque motor.

In contrast to device 300, radiotherapy device 301 depicted in Figures 3c and 3d comprises magnets 362 positioned on an axial face of the rotor 360 (i.e. the magnets face along the axial direction). As can be seen from the figures, the rotor 360 and stator 370 of device 301 overlap in the radial direction and are adjacent to one another in the axial direction. In contrast, the rotor and stator of device 300 (seen in Figures 3a and 3b) overlap in the axial direction and are adjacent to one another in the radial direction. This means that, in the device 301, a magnetic flux path between the magnets 362 of the rotor 360 and the coils of the stator 370 is parallel to the axis of rotation, i.e. along the axial direction. As such, the arrangement of the rotor and stator in the embodiment illustrated in Figure 3c and 3d may be referred to as an axial flux torque motor.

Aside from the difference being that the device 301 depicted in Figure 3c and 3d employs an axial flux torque motor instead of a radial flux torque motor, all other features and advantages of device 301 may be the same as device 300.

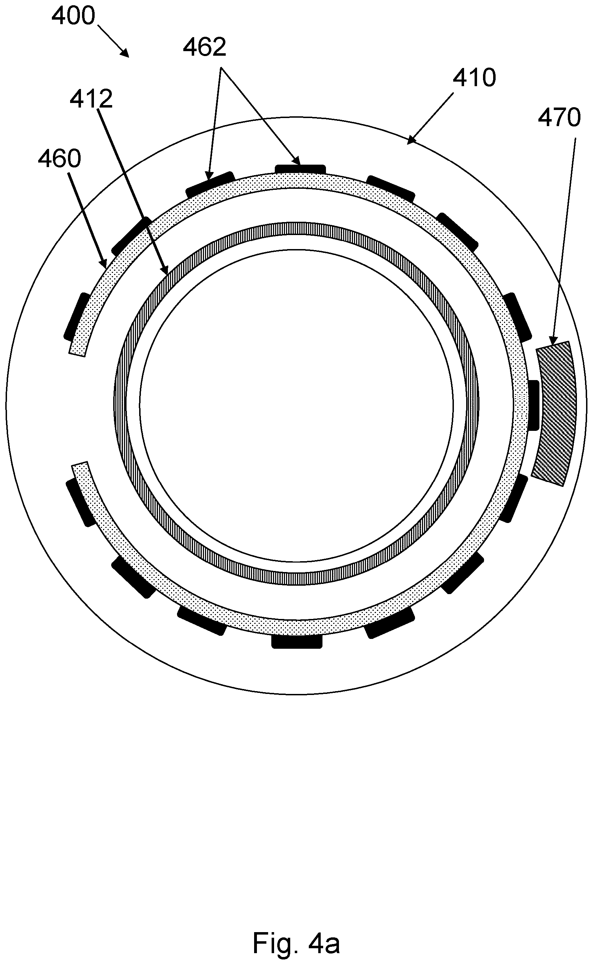

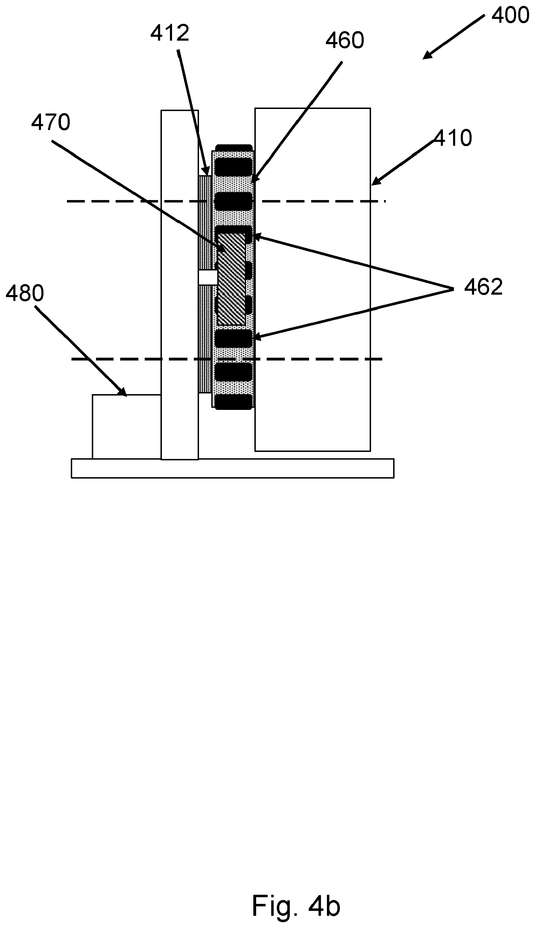

Referring now to Figure 4a and 4b, a radiotherapy device 400 according to another embodiment of the present disclosure is depicted. In this embodiment, the direct drive mechanism used to cause rotation of the gantry is provided by a curved linear motor comprising a rotor 460 and a stator 470. The rotor 460 is a curved track which is mounted to the ring-shaped gantry 410 and follows at least a portion of the curvature of the ring-shaped gantry. The curved track 460 has an arcuate form that corresponds to the curvature of the ring-shaped gantry. Similar to the rotor 360 of radiotherapy device 300, the curved tracked comprises a plurality of magnets 462 spaced along the track and positioned on the outward radial face of the track 460. In some examples the radiotherapy device 400 may also comprise a central bearing 412 that connects the gantry to a stationary part of the device and that supports the weight of the gantry in a cantilever fashion.

The radiotherapy device 400 further comprises a stator 470 which is not fixed to or in physical contact with the gantry 410 or the rotor 460. Instead, as depicted in Figure 4b, the stator 470 is fixed to a stationary part of the device 400 (i.e. a part of the radiotherapy device that is not configured to rotate with the gantry). The stator may also have an arcuate shape that corresponds to the curvature of the curved track of the rotor 460. In some embodiments, the stator 470 comprises a plurality of coils configured to carry an electric current and generate magnetic fields. As would be appreciated by the skilled person, when an electric current is supplied to the coils of the stator, a magnetic field is generated. The stator is positioned to be overlapping with the rotor 460 along the axial direction and adjacent to the rotor along the radial direction, such that magnetic flux path between the rotor and the stator is along the radial direction. The interaction of the magnetic fields produced by the coils and the magnetic fields produced by the magnets 462 on the rotor induces a torque in the rotor which causes rotation of the rotor and in turn the gantry to which the rotor is fixed.

As with the embodiments described above in relation to Figures 3a - 3d, the radiotherapy device 400 may also comprise a power supply unit 480 configured to supply the electrical current to the coils in the stator 470. The power supply unit may further comprise a controller configured to control the output of the power supply unit in the same way as described above in relation to power supply unity 380.

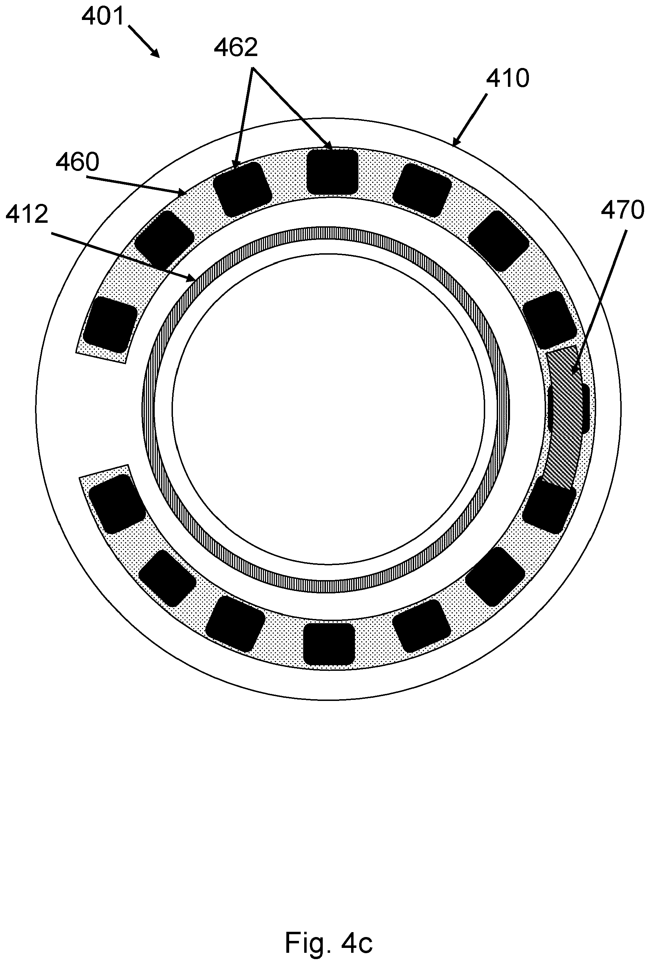

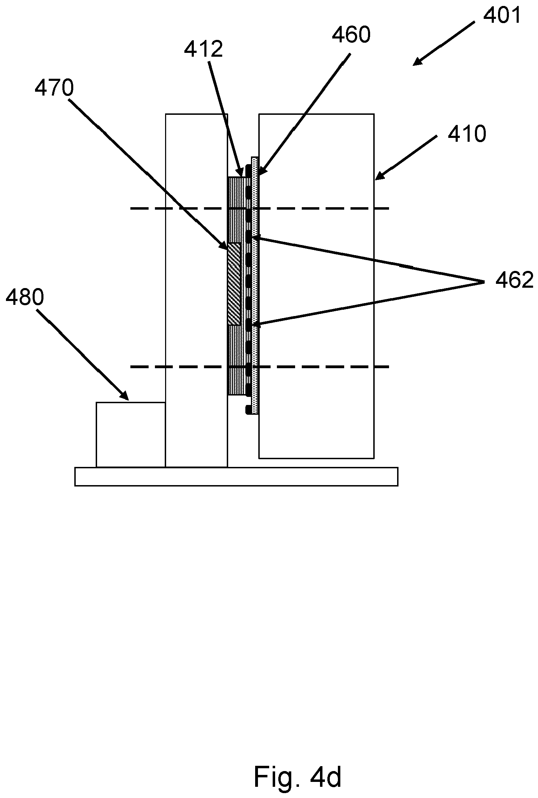

Figures 4c and 4d depict an alternative radiotherapy device 401 that also uses a curved linear motor to cause rotation of the gantry. In contrast to the curved linear motor depicted in Figure 4a and 4b in which the magnetic flux path between the rotor and the stator is along the radial direction, device 401 depicted in Figures 4c and 4d has a rotor and stator arranged such that the magnetic flux path between the two is along the axial direction. In more detail, radiotherapy device 401 comprises a curved track rotor 460 that has an arcuate form corresponding to the curvature of the gantry. The curved track comprises a plurality of magnets spaced along the track and positioned on the axial face of the track such that the magnets face along the axial direction. The stator 470 is not fixed to or in physical contact with the gantry or the rotor but is fixed instead to a stationary part of the device 401. The stator may also have an arcuate shape corresponding to the arcuate curvature of the rotor track 460. Similar to the embodiments described above, the stator may comprise a plurality of coils configured to generate magnetic fields in the same manner as described above in relation to Figures 3a to 4b. The arrangement of the rotor 460 and the stator 470 in this embodiment are such that the rotor and stator overlap in the radial direction (as shown in Figure 4c) and are adjacent in the axial direction (as shown in Figure 4d). As such, the magnetic flux path between the rotor and stator is along the axial direction.

As with the embodiments described above in relation to Figures 3a - 4b, the radiotherapy device 401 may also comprise a power supply unit 480 configured to supply the electrical current to the coils in the stator 470. The power supply unit may further comprise a controller configured to control the output of the power supply unit.

It will be appreciated that in some embodiments of the present disclosure, the components of the rotor and stator as described above may be switched and the same purpose of causing rotation of the gantry would still be achieved. That is, the rotor fixed to the gantry may comprise coils to which current is supplied, whilst the stator comprises the permanent magnets (such as magnets 362 and 462). In these embodiments, a current is supplied to the coils of the rotor, which generates a magnetic field around the coils. The magnetic field interacts with magnetic fields generated by the permanent magnets of the stator to induce a torque in the rotor, thereby causing rotation of the gantry.

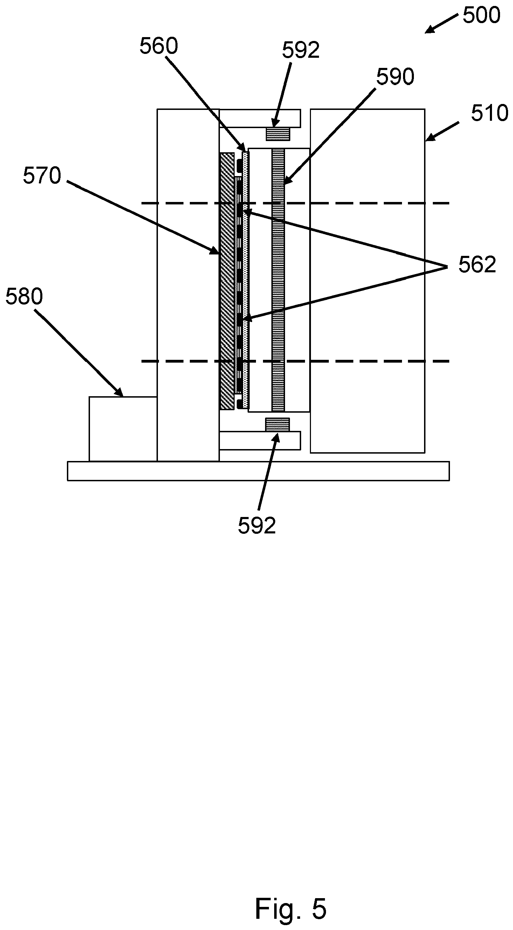

Reference is now made to Figure 5, which depicts a radiotherapy device 500 according to some embodiments of the invention. The device 500 comprises an axial torque motor configured to cause rotation of the gantry, comprising a ring-shaped rotor 560 fixed to the gantry 510 and a corresponding ring-shaped stator 570 adjacent to the rotor along the axial direction. The torque motor of device 500 is therefore the same as the motor used in the device depicted in Figure 3c and 3d and is described in more detail above in relation to those figures.

The radiotherapy device 500 further comprises an encoder strip 590 would around a drum portion of the gantry. The encoder strip may be wound around the entire circumference of the drum of the gantry 510, such that the encoder strip can be used to provide feedback on the gantry rotation position. The controller can use the feedback from the encoder strip to control rotation (i.e. rotational position and speed) of the gantry. As would be appreciated by the skilled person, the encoder strip can be magnetic, optical, capacitive or any other suitable type of encoder strip that is suitable for determining the rotary position of the gantry. In order to read the encoder strip to provide the feedback described above, the device 500 further comprises one or more encoder reader heads 592, also called encoder sensors. The encoder sensor(s) 592 are fixed to a stationary portion of the device such as a base and are positioned adjacent to the encoder strip 590. The encoder strip 590 therefore moves relative to the encoder sensors 592 as the gantry 510 rotates. The encoder sensors 592 are positioned close enough to the encoder strip 590 so as to be able to read the encoder strip and send signals to a rotational measurement system. The rotational measurement system may be comprised within the controller 580 and is configured to measure the rotational position of the gantry 510 based on the readings of the encoder sensor(s) 592. The rotational position of the gantry measurement can then be used by the controller to control the rotation of the gantry. Additionally, the rotational position measurement can be used to inform treatment delivery and imaging.

In some embodiments, the radiotherapy device comprises at least two encoder sensors 592. At the position on the gantry drum where the two opposite ends of the encoder strip meet, a gap or break in the encoder strip may exist. A reading taken from an encoder sensor at this gap or break may result in inaccurate position measurements. To address this issue, at least two encoder sensors may be used and may be positioned such that, for any given rotational position of the gantry, at least one of the at least two encoder sensors will not be adjacent to the gap in the encoder strip. As depicted in the particular embodiment of Figure 5, two diametrically opposite encoder sensors 592 are used. This means that accurate readings can be acquired for all angles of rotation. It would be appreciated that the reader heads may be positioned adjacent to one another, diametrically opposite one another as depicted in the Figure 5, or at any intermediate position.

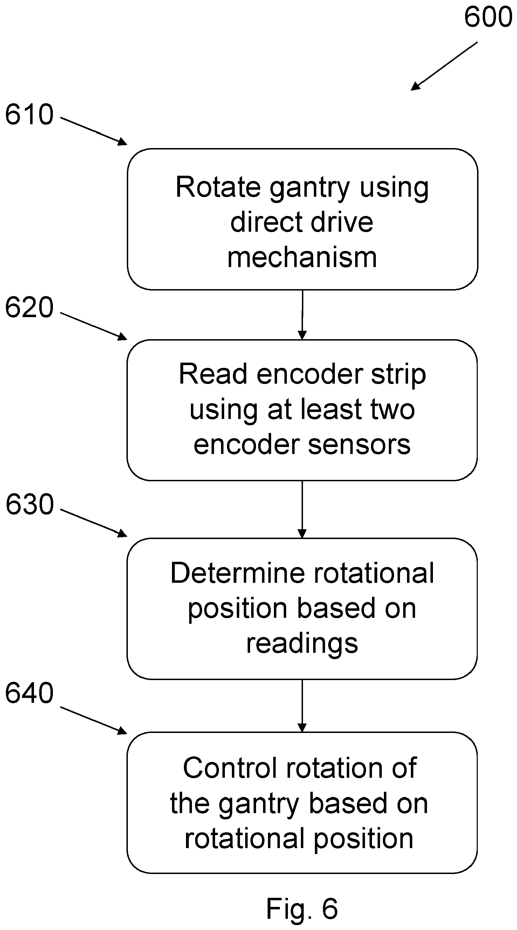

Figure 6 depicts a flowchart of a method 600 according to the present disclosure. The method comprises a step 610 of rotating a gantry of a radiotherapy device using a direct drive mechanism. The radiotherapy device many be similar to devices 300, 301, 400, 401 or 500 as described above. The direct drive mechanism may comprise be a torque motor or a curved linear motor, such as those described above in relation to Figures 3a - 4d. Rotating the gantry using the direct drive mechanism may comprise using a controller to control the supply of current to the stator of the direct drive mechanism. The controller may cause a power supply to supply current to the electrical coils, which generates a magnetic field around those coils. The generated magnetic fields interact with magnetic fields produced by permanent magnets on a rotor of the direct drive mechanism, and the interaction of the generated and permanent magnetic fields induces a torque in the rotor. The rotor is fixed to and coaxial with the gantry such that the torque induced in the rotor causes the gantry to rotate.

The method may optionally further comprise a step 620 of reading an encoder strip that is wound around a drum of the gantry. The encoder strip provides information on the rotational position of the gantry, and encoder sensors fixed to a stationary part of the gantry are positioned to be able to read the encoder strip would around the gantry as the gantry rotates. At least two encoder sensors may be used so that accurate readings of the encoder strip may be taken at all gantry positions, so that any gap between opposite ends of the encoder strip does not lead to inaccurate readings.

The method may optionally further comprise a step 630 of determining the rotational position of the gantry based on the readings acquired by the encoder sensors. The encoder sensors may send a signal to a rotational measurement system that is able to determine the rotational position of the gantry based on the readings from the encoder sensors.

In some embodiments, the method may further comprise a step 640 of controlling rotation of the gantry based on the determined rotational position of the gantry. This may involve causing the gantry to cease movement, to decelerate, or to initiate rotation based on the current rotational position.

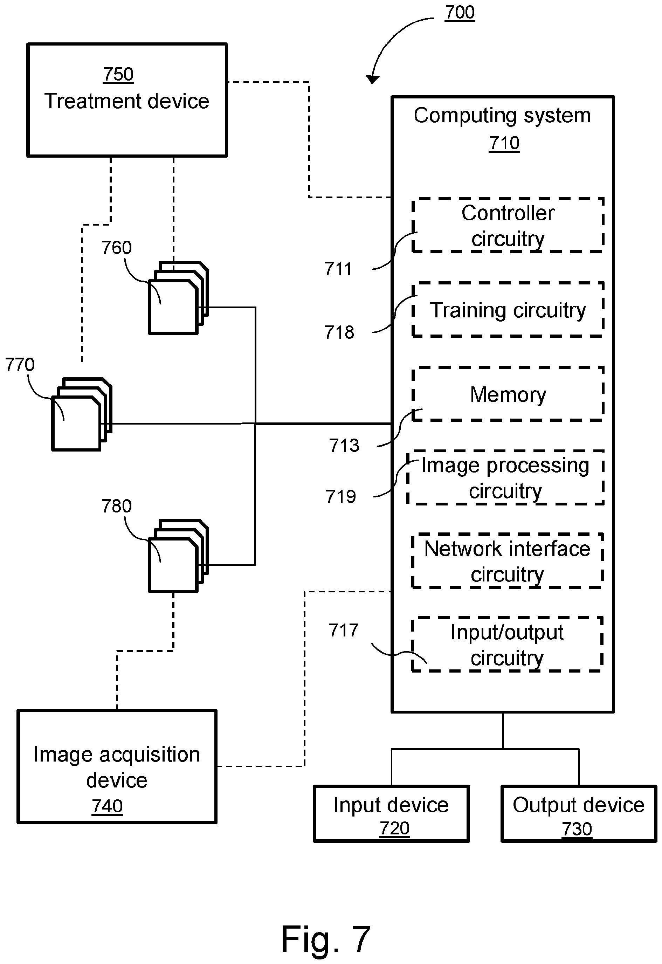

Figure 7 illustrates a block diagram of one implementation of a radiotherapy system 700. The radiotherapy system 700 comprises a computing system 710 within which a set of instructions, for causing the computing system 710 to perform any one or more of the methods discussed herein, may be executed.

The computing system 710 shall be taken to include any number or collection of machines, e.g. computing device(s), that individually or jointly execute a set (or multiple sets) of instructions to perform any one or more of the methods discussed herein. That is, hardware and/or software may be provided in a single computing device, or distributed across a plurality of computing devices in the computing system. In some implementations, one or more elements of the computing system may be connected (e.g., networked) to other machines, for example in a Local Area Network (LAN), an intranet, an extranet, or the Internet. One or more elements of the computing system may operate in the capacity of a server or a client machine in a client-server network environment, or as a peer machine in a peer-to-peer (or distributed) network environment. One or more elements of the computing system may be a personal computer (PC), a tablet computer, a set-top box (STB), a Personal Digital Assistant (PDA), a cellular telephone, a web appliance, a server, a network router, switch or bridge, or any machine capable of executing a set of instructions (sequential or otherwise) that specify actions to be taken by that machine.

The computing system 710 includes controller circuitry 711 and a memory 713 (e.g., read-only memory (ROM), flash memory, dynamic random access memory (DRAM) such as synchronous DRAM (SDRAM) or Rambus DRAM (RDRAM), etc.). The memory 713 may comprise a static memory (e.g., flash memory, static random access memory (SRAM), etc.), and/or a secondary memory (e.g., a data storage device), which communicate with each other via a bus (not shown).

Controller circuitry 711 represents one or more general-purpose processors such as a microprocessor, central processing unit, accelerated processing units, or the like. More particularly, the controller circuitry 711 may comprise a complex instruction set computing (CISC) microprocessor, reduced instruction set computing (RISC) microprocessor, very long instruction word (VLIW) microprocessor, processor implementing other instruction sets, or processors implementing a combination of instruction sets. Controller circuitry 711 may also include one or more special-purpose processing devices such as an application specific integrated circuit (ASIC), a field programmable gate array (FPGA), a digital signal processor (DSP), network processor, or the like. One or more processors of the controller circuitry may have a multicore design. Controller circuitry 711 is configured to execute the processing logic for performing the operations and steps discussed herein.

The computing system 710 may further include a network interface circuitry 718. The computing system 710 may be communicatively coupled to an input device 720 and/or an output device 730, via input/output circuitry 717. In some implementations, the input device 720 and/or the output device 730 may be elements of the computing system 710. The input device 720 may include an alphanumeric input device (e.g., a keyboard or touchscreen), a cursor control device (e.g., a mouse or touchscreen), an audio device such as a microphone, and/or a haptic input device. The output device 730 may include an audio device such as a speaker, a video display unit (e.g., a liquid crystal display (LCD) or a cathode ray tube (CRT)), and/or a haptic output device. In some implementations, the input device 720 and the output device 730 may be provided as a single device, or as separate devices.

Image acquisition device 740 may be configured to perform positron emission tomography (PET), computed tomography (CT), magnetic resonance imaging (MRI), ... <do we have some boilerplate for this already? If so, might be best to insert it here>.

Image acquisition device 740 may be configured to output image data 780, which may be accessed by computing system 710. Treatment device 750 may be configured to output treatment data 760, which may be accessed by computing system 710.

Computing system 710 may be configured to access or obtain treatment data 760, planning data 770 and/or image data 780. Treatment data 760 may be obtained from an internal data source (e.g. from memory 713) or from an external data source, such as treatment device 750 or an external database. Planning data 770 may be obtained from memory 713 and/or from an external source, such as a planning database. Planning data 770 may comprise information obtained from one or more of the image acquisition device 740 and the treatment device 750.

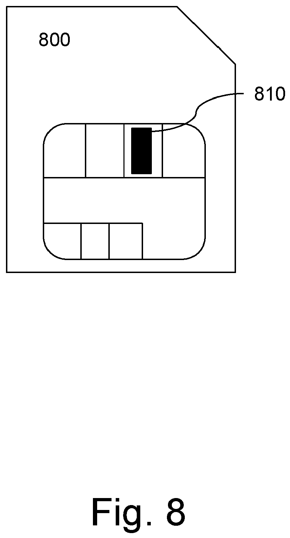

The various methods described above may be implemented by a computer program. The computer program may include computer code (e.g. instructions) 810 arranged to instruct a computer to perform the functions of one or more of the various methods described above. The steps of the methods described above may be performed in any suitable order. The computer program and/or the code 810 for performing such methods may be provided to an apparatus, such as a computer, on one or more computer readable media or, more generally, a computer program product 800)), depicted in Figure 8. The computer readable media may be transitory or non-transitory. The one or more computer readable media 800 could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD. The instructions 810 may also reside, completely or at least partially, within the memory 713 and/or within the controller circuitry 711 during execution thereof by the computing system 710, the memory 713 and the controller circuitry 711 also constituting computer-readable storage media.

In an implementation, the modules, components and other features described herein can be implemented as discrete components or integrated in the functionality of hardware components such as ASICS, FPGAs, DSPs or similar devices.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may comprise a special-purpose processor, such as an FPGA or an ASIC. A hardware component may also include programmable logic or circuitry that is temporarily configured by software to perform certain operations.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

Unless specifically stated otherwise, as apparent from the following discussion, it is appreciated that throughout the description, discussions utilizing terms such as " receiving", "determining", "comparing ", "enabling", "maintaining," "identifying," [CHANGE LANGUAGE AS APPROPRITE TO SPECIFICATION] or the like, refer to the actions and processes of a computer system, or similar electronic computing device, that manipulates and transforms data represented as physical (electronic) quantities within the computer system's registers and memories into other data similarly represented as physical quantities within the computer system memories or registers or other such information storage, transmission or display devices.

It is to be understood that the above description is intended to be illustrative, and not restrictive. Many other implementations will be apparent to those of skill in the art upon reading and understanding the above description. Although the present disclosure has been described with reference to specific example implementations, it will be recognized that the disclosure is not limited to the implementations described, but can be practiced with modification and alteration within the spirit and scope of the appended claims. Accordingly, the specification and drawings are to be regarded in an illustrative sense rather than a restrictive sense. The scope of the disclosure should, therefore, be determined with reference to the appended claims, along with the full scope of equivalents to which such claims are entitled.

## Claims

1. A radiotherapy apparatus comprising a rotatable ring-shaped gantry and a direct drive mechanism, wherein the direct drive mechanism is configured to cause rotation of the gantry.

2. The radiotherapy apparatus of claim 1, wherein the direct drive mechanism is configured to transmit torque directly to the gantry without intermediate transmission components.

3. The radiotherapy apparatus of claim 1 or 2, further comprising a base, and wherein the gantry is configured to rotate relative to the base, and wherein the direct drive mechanism comprises a rotary portion fixed to the gantry and a stationary portion fixed to the base.

4. The radiotherapy apparatus of any preceding claim, wherein the direct drive mechanism comprises a ring-shaped torque motor.

5. The radiotherapy apparatus of any of claims 1 to 3, wherein the direct drive mechanism comprises a curved linear motor.

6. The radiotherapy apparatus of any preceding claim, wherein the gantry comprises a drum and an encoder strip wound around the drum of the gantry.

7. The radiotherapy apparatus of claim 6, further comprising at least one encoder sensor and a rotational measurement system, wherein the encoder sensor is configured to read the encoder strip and wherein the rotational measurement system is configured to measure rotational position of the gantry based on the reading of the encoder sensor.

8. The radiotherapy apparatus of claim 7, comprising two or more encoder sensors configured to read the encoder strip.

9. The radiotherapy apparatus of claim 7 or 8, wherein the at least one encoder sensor is fixed to the base or the stationary portion of the direct drive mechanism.

10. A method of rotating a gantry of a radiotherapy apparatus, the method comprising causing rotation of the gantry using a direct drive mechanism.

11. The method of claim 10, wherein the gantry comprises a drum and an encoder strip wound around the drum, and wherein the method comprises reading the encoder strip using two or more encoder sensors.

12. The method of claim 11, further comprising determining a rotational position of the gantry based on the readings from the two or more encoder sensors.

13. The method of claim 12, further comprising controlling rotation of the gantry based on the determined of the rotational position of the gantry.

14. The method of claim 12 or 13, further comprising controlling a radiotherapy treatment based on the determined rotational position of the gantry.

15. A computer-readable medium carrying instructions that, when executed by one or more processors, cause the one or more processors to carry out the method of any one of claims 10 to 14.
